Europäisches Patentamt

European Patent Office

Office européen des brevets

(11) Publication number: 0 318 098
A1

## EUROPEAN PATENT APPLICATION

(21) Application number: 88202622.2

(22) Date of filing: 22.11.88

(51) Int. Cl.4: C02F 3/28

(30) Priority: 26.11.87 NL 8702843

(43) Date of publication of application:
31.05.89 Bulletin 89/22

(84) Designated Contracting States:
AT BE CH DE ES FR GB GR IT LI LU NL SE

(71) Applicant: DHV Raadgevend
Ingenieursbureau BV
Laan 1914 nr. 35
NL-3818 EX Amersfoort(NL)

(72) Inventor: Sayed, Sameh Khalil Ibrahim
Groen van Prinstererstraat 129
NL-6702 CR Wageningen(NL)
Inventor: Schellinkhout, Aris
Emmapark 9
NL-6701 CA Wageningen(NL)

(74) Representative: Kupecz, Arpad et al
Octrooibureau Los en Stigter B.V. Postbox
20052
NL-1000 HB Amsterdam(NL)

(54) Process and apparatus for the anaerobic purification of waste water.

(57) The invention relates to a process and an apparatus for the anaerobic purification of waste water.

According to the invention the waste water is purified in two steps.

In the first step the waste water is for the major part purified in a first series of reactors (2, 3) by biofiltration, entrapment and sludge stabilization. In the second step (4) the effluent derived from the first series of reactors is further purified to the required grade of purity where the impurities are converted into methane.

Usually the first series of reactors is operated intermittently, whereas the second series of reactors is operated continuously.

However it is also possible to operate the first series of reactors continuously maintaining good overall process results.

fig.1

## Process and apparatus for the anaerobic purification of waste water.

The present invention relates to a process for the anaerobic purification of waste water, as well as to an apparatus for carrying out this process.

Such a process for the anaerobic purification of waste water is known. According to the known process the purification of the waste water takes place through a single-step system, which is continuously operated. Such known highly loaded anaerobic purification systems are mainly developed for waste water containing very low concentrations of coarse suspended and colloidal material. The known systems are only partly capable of purifying complex waste water, i.e. waste water containing a large fraction of coarse suspended and colloidal material, such as domestic waste water and agroindustrial waste water.

The above mentioned single-step systems may be subdivided into systems which make use of a granular sludge reactor or systems, which make use of a digested sewage sludge reactor.

A single-step system, whereby use is made of granular sludge reactor is described in A.W.A. de Man, P.C. Grin, R.E. Roersma, K.C.F. Grolle and G. Lettinga (1986), Aerobic treatment of municipal waste water at low temperatures.

This system has the disadvantage that coarse suspended material is rinsed out, whereby a low purification efficiency is obtained. Furthermore adsorption of the colloidal material takes place at the sludge bed, whereby the specific methanogenic activity of the sludge is reduced, which results in a low applicable load (organic and hydraulic).

A single-step system, whereby used is made of digested sewage sludge reactor, is known from G. Lettinga, A.F.M. van Velsen, S.W. Hobma, W. de Zeeuw and A. Klapwijk (1980), Use of the upflow sludge blanket (USB) reactor concept for biological waste water treatment, especially for anaerobic treatment. Biotechnology & Bioengineering, 22, 699-734, whereby because of accumulation as a result of entrapping mechanism of coarse suspended material and accumulation as a result of adsorption mechanism of colloidal material there is a great difference between the purification efficiency and the COD-conversion into methane. Furthermore there occurs an unfavourable formation of a floating layer as well as an unsatisfactory low sludge stabilization, whereas the applicable load is low. Further practice has shown that as a result of continuous accumulation of coarse suspended and colloidal material the process becomes unstable and will eventually lead to sudden undesired foam formation, sludge floatation and the total disappearance of the active biomass from the system.

The present invention aims at providing a process, with which the above mentioned disadvantages of the known single-step system will be eliminated in an efficient way.

For this purpose the invention provides a process for the anaerobic purification of waste water, characterized in that waste water having a high content of coarse suspended and colloidal material is purified in two steps, whereby in the first step the waste water is supplied into a reactor of a first series of reactors comprising at least two reactors, in which reactor the waste water is biologically filtered and sludge stabilization takes place, and the biogas formed thereby is discharged to a gas holder and the effluent is led to a second series of reactors comprising at least one reactor, whereas the other reactor of the first series of reactors is regenerated and in the second series of reactors the organic impurities left behind in the effluent of the one reactor undergo an anaerobic conversion into methane gas, which gas is subsequently supplied to the gas holder, whereas the effluent originating from the second series of reactors, which is practically free from organic impurities, is discharged.

The process according to the invention, which is a two-step process, is in particular suitable for the anaerobic purification of waste water having the following characteristics: The contaminations in the waste water are biologically decomposable. The waste water is a complex, i.e. it contains a high content of coarse suspended and colloidal material. The temperature of the waste water to be treated may vary, i.e. that the temperature thereof generally is within the psychrophilic and thermophilic range. The composition of the waste water varies highly with respect to COD, BOD, coarse suspended and colloidal material (COD = chemical oxygen demand; BOD = biochemical oxygen demand).

Furthermore the waste water may contain different substances, such as fat and proteins, which may result in the formation of a floating layer.

Examples of waste water, which fulfils the abovementioned characteristics are: waste waters derived from the sugar industry, domestic waste water, slaughter-houses, meat processing industry, potato and starch processing industry, breweries, paper industry, fish and mussel industry.

As mentioned before the process of the invention is a so-called two-step process, that is that said process is based on a combination of two series of anaerobic reactors, wherein the first series of reactors is generally operated intermittently, whereas the second series is operated continuously. However the first series of reactors also can be operated continuously.

It should be understood that the first series of reactors acts as a biological filter, combined with sludge stabilization, whereas the second series of reactors acts according to the principle of the anaerobic methane digesting process.

The temperature of the influent waste water in the first and second series of reactors is within the range of 10-60° C, whereas in the other reactors of the first series, wherein regeneration occurs, the temperature is within 30-60° C. In the first series of reactors a fine flocculent inocculation sludge, preferably digested sewage sludge is used as inocculation sludge, derived from for instance the conventional sewage water purification. The fine flocculent sludge is able to entrap the coarse suspended and colloidal material, that is to filtrate the same, so that the effluent, which leaves the reactor, is essentially freed from all coarse suspended and colloidal material.

The said digested sewage sludge settles very slowly.

In the second series of reactors as inocculation sludge a granular sludge is used having a diameter of 0,5 - 5 mm, which granules are relatively heavy and therefore they settle quickly.

As mentioned before the first series of reactors is generally operated intermittently, wherein during operation of one reactor the other is being regenerated, wherein when in the one reactor the sludge together with the entrapped coarse suspended and colloidal material has achieved a certain level, the introduction of the influent to the one reactor is stopped and it is introduced into the other reactor which is now regenerated, whereafter that one reactor is regenerated and the effluent from the first series of reactors is continuously supplied into the second series of reactors.

The regeneration of the first series of reactors occurs by recirculation of the biogas from the gas holder and/or by the recirculation of the effluent originating from the first or from the second series of reactors.

According to another suitable embodiment of rhe present invention the process is characterized in that the first series of reactors is operated continuously, wherein one of the first series of reactors during operation is continuously regenerated, whereas into the other reactor both the waste water and the regenerated sludge continously are introduced and the effluent from the other reactor continuously is introduced into the second series of reactors.

According to an advantageous embodiment of the process of the invention the first series of reactors is operated at an upward velocity of 0.1 - 2.0 m/hour, whereas the second series of reactors is operated at an upward velocity of 0.1 - 10.0 m/hour. In the practice of the invention the residence time of the liquid is 6 - 36 hours in each of the reactors of both series.

Furthermore the invention relates to an apparatus for carrying out the process of the invention, which appa ratus is characterized in that it comprises a first series of reactors with at least two reactors and a second series of reactors with at least one reactor, whereby each of the first series of reactors is provided with an influent supply for waste water with a high content of coarse suspended and colloidal material for supplying this waste water at the bottom of the reactor, which reactor furthermore comprises fine flocculent filter material, the upper side of each of the reactors is connected with the bottom side of the second series of reactors through a first conduit for supplying the effluent originating from the first series of reactors and with a gas holder through a second conduit for discharging the formed biogas to the gas holder, whereas the reactor of the second series of reactors at the upper side comprises a conduit for discharging the effluent and a conduit for discharging the formed biogas to the gas holder, whereas the discharge conduit for the second effluent is connected with a conduit for the recirculation of the effluent in respectively the one and the other reactor of the first series of reactors, whereby finally the gas holder at the upper side comprises a conduit for recirculating the biogas in the first and second reactor of the first series of reactors and a conduit for discharging the biogas to the consumer.

The advantages of the present two-step anaerobic system compared with the conventional one-step anaerobic system are as follows:

The two-step system offers a high process stability and process flexibility, that is that the system is very much able to manage organic peak loads. Furthermore the system according to the invention is able to manage continuously a high load because of the intermitting operation of the first series of reactors. However it appeared that by continuous operation of the first series of reactors similar satisfactory results may be obtained. The system saves furthermore energy compared with other anaerobic one-step systems, which are continuously operated at a temperature of 30° C. In the event that waste water is treated with a temperature of 10-30° C the contents of the first series of reactors is to be heated only during some weeks up to 30° C instead of the continuous heating of the waste water, which results in a significant energy saving. Furthermore an optimum energy gain is obtained according to the invention in the form of biogas.

It should be understood that the present two-step system enables the complete treatment of a complex waste water, because maximally 90% of both the coarse suspended material and colloidal material is digested in the first series of reactors.

Furthermore there is about 80% BOD-removal in the first series of reactors, whereas furthermore in the second series of reactors during a feed stop period a complete sludge stabilization occurs (that is a complete conversion into methane of the accumulated coarse suspended and colloidal material). Furthermore there is an 80% BOD-removal and conversion into methane of the effluent from the first series of reactors (that is the dissolved fraction of the waste water) in the second series of reactors (granular sludge reactor).

Furthermore as advantage may be mentioned that the present process requires low investment costs, because the methane reactor is smaller than in the case of the one-step systems.

Besides the known one-step anaerobic purification system other conventional aerobic systems are applied too.

The advantages of the present two-step anaerobic system compared with the conventional aerobic systems are: low investment costs and annual costs, because according to the invention use is made of a combination of high purification capacity with a small reactor volume, which means that a small ground surface is required.

Furthermore according to the present two-step system no oxygen is required, which means that there is ample opportunity of energy saving. Furthermore according to the invention there is a significantly increased biogas production, whereas the system does not smell, because the reactors are closed vessels.

The invention will be explained in more detail by referring to the drawings, to which the invention is not restricted.

Fig. 1 represents a preferred embodiment of the apparatus according to the invention for carrying out the present process, wherein the first series of reactors is operated intermittently.

Fig. 2 shows another favourable embodiment of the apparatus according to the invention, wherein the first series of reactors is continuously operated.

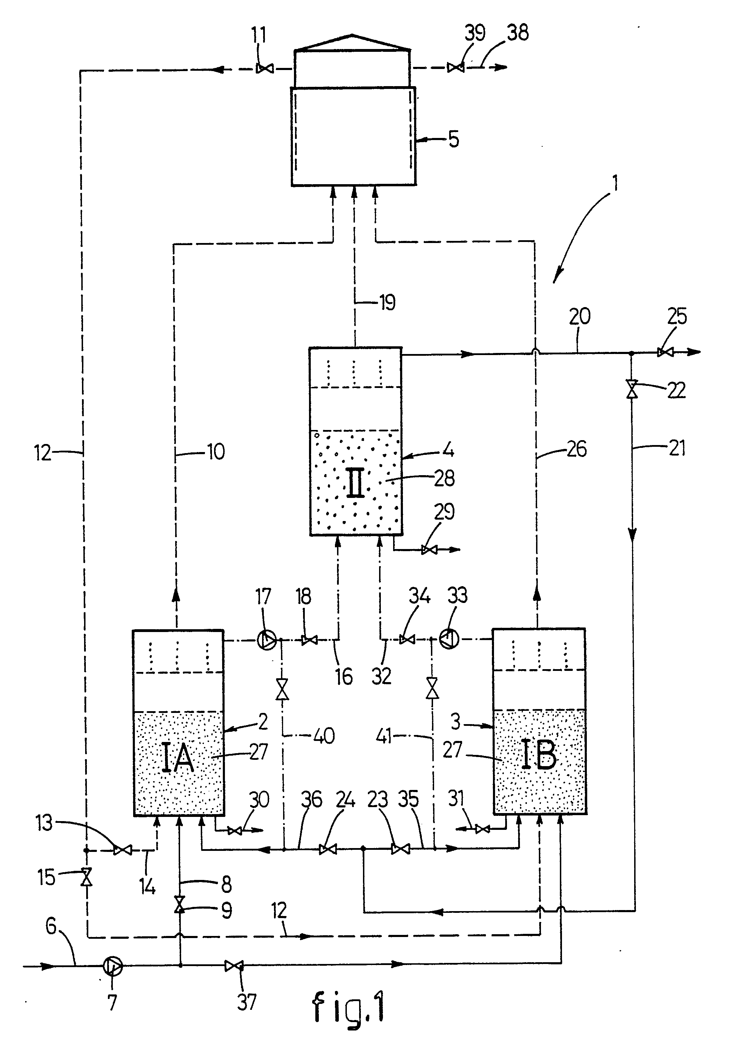

Fig. 1 shows the apparatus 1 according to the invention, which apparatus is provided with a first series of reactors 2 and 3, IA and IB respectively. In this preferred embodiment of the apparatus according to the invention the first series of reactors comprises two anaerobic reactors, although more than two reactors may be used too.

Further the apparatus according to the invention 1 comprises a second series of reactors 4, which in the preferred embodiment only comprises one reactor II.

Then the apparatus according to the invention is provided with a gas holder 5 for storage of the biogas which is formed in the first series and second series of reactors.

The first series of reactors are inocculated with a fine flocculent sludge 27 having a large specific surface, which sludge is light and therefore it remains longer in suspension. The fine flocculent sludge is preferably a digested sewage sludge, which is for instance originating from the conventional sewage water purification devices. The primary function of the fine flocculent sludge 27 in the first series of reactors IA and IB is the biological filtration of coarse suspended and colloidal material present in the waste water, which material is entrapped in sludge material.

Reactor II of the second series of reactors contains as inocculation sludge material a granular sludge 28 having a diameter of 0,5 - 5 mm. This sludge material has a relatively low specific surface. Such a sludge settles quickly. This sludge is for instance derived from the usual anaerobic purification devices.

During operation the reactors IA and IB of the first series of reactors are intermittently operated with the waste water to be purified, which means that in a practical embodiment of the process the waste water is introduced into the reactor IA via conduit 6, pump 7, conduit 8 and valve 9, which is now open, whereas valve 37 in conduit 6 is closed, so that reactor IB is not fed. The coarse suspended and colloidal material present in the waste water is entrapped by the digested sewage sludge with high specific surface, whereupon the effluent leaves the reactor IA through the top via conduit 16, pump 17 and valve 18, which is now open, which effluent is introduced in reactor II of the second series of reactors.

The temperature of the influent waste water, which enters the reactor IA, may vary between 10°C and 60°C, that is that the waste water having a considerable temperature variation successfully may be treated according to the present invention.

The effluent, which is freed from coarse suspended and colloidal material, is further purified in reactor II by anaerobical conversion of the organic materials into methane gas.

After passing reactor II the effluent, which leaves the reactor at the top, appears to be sufficiently pure, so that this effluent may be discharged via conduit 20 and valve 25. The biogases as formed in the reactors IA and II respectively are discharged to a gas container 5 at the top of the reactor via conduits 10 and 19 respectively. From the gas holder 5 the biogas may be provided to a consumer via conduit 38 and valve 39.

After a certain time, for instance some weeks, the sludge level in reactor IA increases and will be saturated so that the reactor does not function any more as biological filter for the coarse suspended and colloidal material. This is the moment that reactor IA has to be regenerated. Prior to the regeneration the introduction of the influent to reactor IA is stopped by closing valve 9, whereas valve 37 is opened in order to introduce the influent waste water into the regenerated reactor IB. Reactor IB operates similarly as reactor IA, wherein the formed biogas is discharged via conduit 26 to the gas holder 5 and the effluent at the top through conduit 32, pump 33 and valve 34 is discharged to reactor II, which operates continuously.

The regeneration of reactor IA, in which event valves 9 and 18 are closed, occurs by the introduction of recirculation gas from the gas holder 5 via conduit 12, wherein valve 15 is closed and the valves 11 and 13 are opened. The recirculation gas is introduced with such a velocity, that the reactor content is maintained in a fluidized condition in order to promote the anaerobic conversion of the organic material into methane gas.

The regeneration of reactor IA may also occur by means of the effluent, leaving the second reactor, in which case the effluent is introduced into reactor IA via conduit 21 and conduit 36. It is self-explanatory that during the regeneration of the reactor IA valve 23 is closed.

The regeneration may occur by simultaneous recirculation of both the biogas and the effluent.

It is noted that the conduits 16 en 36 on the one hand and the conduits 32 and 35 on the other hand may be connected to each other by means of recirculation conduits 40 and 41 for the recirulation of the contents of the reactors IA and IB.

During the regeneration of reactor IA the contents of the reactor is maintained at a temperature between 30° C and 60° C in order to ensure a rapid conversion of accumulated coarse suspended and colloidal material into methane in the reactor. After a certain time the reactor IA is regenerated and is ready again for application for the filtration of the influent waste water, as soon as reactor IB is saturated. Then the process is repeated wherein reactor IA is fed with the influent waste water, whereas reactor IB is regenerated in the way as disclosed before.

Finally the sludge of the reactors IA, IB and II respectively may be discharged via conduits 30, 31 and 29.

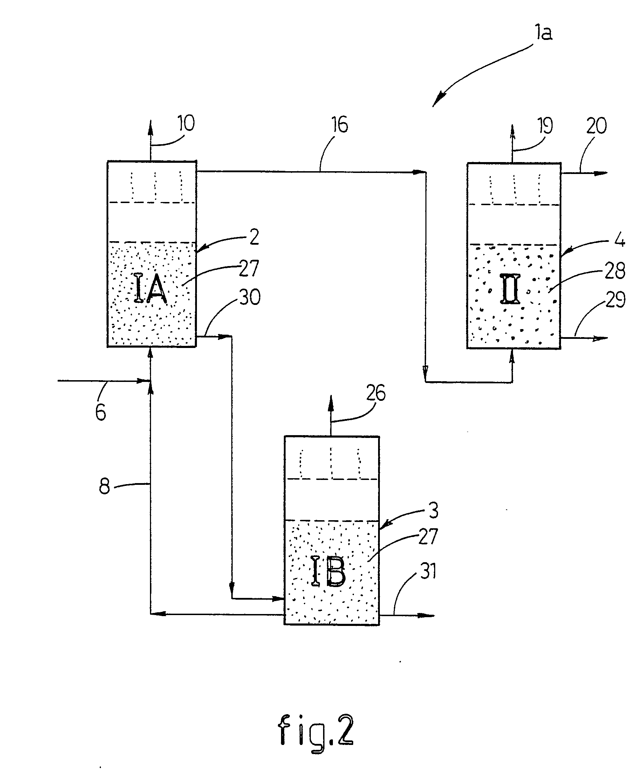

Fig. 2 shows the first and second series of reactors of another favourable embodiment of the apparatus 1a according to the invention, wherein the conduits 10, 26 and 19 are connected with a similar gas holder as in fig. 1.

A special aspect of this embodiment is the fact that the reactors IA and IB are operated continuously instead of intermittently such as in fig. 1.

In reactor IB the sludge is supplied continuously via conduit 30 from reactor IA. Then the sludge is subjected to a regeneration process and is continuously supplied via conduit 8 to reactor IA. For the remaining the process occurs similarly as in fig. 1.

The meaning of the reference figures is the same as in fig. 1.

## Claims

1. A process for the anaerobic purification of waste water, **characterized** in that waste water with a high content of coarse suspended and colloidal material is purified in two steps, whereby in the first step the waste water is supplied into a reactor of a first series of reactors comprising at least two reactors, in which reactor the waste water is biologically filtered and sludge stabilization takes place, and the biogas formed thereby is discharged to a gas holder and the effluent is led to a second series of reactors comprising at least one reactor, whereas the other reactor of the first series of reactors is regenerated and in the second series of reactors the organic impurities left behind in the effluent of the one reactor undergo an anaerobic conversion into methane gas, which gas is subsequently supplied to the gas holder, whereas the effluent originating from the second series of reactors, which is practically free from organic impurities, is discharged.

2. The process according to claim 1, **characterized** in that the temperature of the influent waste water in the first and second series of reactors is about 10-60° C, whereas with the other reactors of the first series, in which regeneration takes place, the temperature is about 30-60° C.

3. The process according to claims 1 or 2, **characterized** in that in the first series of reactors as inocculation sludge digested sewage sludge is used, whereas in the second series of reactors as inocculation sludge granular sludge is applied.

4. The process according to claims 1 - 3, **characterized** in that the first series of reactors is operated intermittently, whereby during operation of the one reactor the other reactor is regenerated, whereby when in the one reactor the sludge with roughly suspended and colloidal material entrapped therein has reached a certain level, the supply of the influent to that one reactor is stopped and is supplied into the other presently regenerated reactor, whereafter that one reactor is regenerated and the effluent from the first series of reactors is continuously supplied into the second series of reactors.

5. The process according to claim 4, **characterized** in that the first series of reactors is regenerated by recirculation of the biogas from the gas holder and/or by the recirculation of the effluent originating from the first or from the second series of reactors.

6. The process according to claims 1 - 3, **characterized** in that the first series of reactors is operated continuously, wherein one reactor of the first series of reactors during operation is continuously regenerated, whereas into the other reactor both the waste water and the generated sludge continuously are introduced and the effluent from the other reactor continuously is introduced into the second series of reactors.

7. Process according to claims 1 - 6, **characterized** in that the first series of reactors is operated at an upward velocity of 0.1 - 2.0 meters/hour, whereas the second series of reactors is operated at an upward speed of 0.1 - 10.0 meters/hour.

8. Apparatus for carrying out the process according to any one of the preceding claims, **characterized** in that the apparatus comprises a first series of reactors with at least two reactors and a second series of reactors with at least one reactor, whereby each of the first series of reactors is provided with an influent supply for waste water with a high content of coarse suspended and colloidal material for supplying this waste water at the bottom of the reactor, which reactor furthermore comprises fine flocculent filter material, the upper side of each of the reactors is connected with the bottom side of the second series of reactors through a first conduit for supplying the effluent originating from the first series of reactors and with a gas holder through a second conduit for discharging the formed biogas to the gas holder, whereas the reactor of the second series of reactors at the upper side comprises a conduit for discharging the effluent and a conduit for dis charging the formed biogas to the gas holder, whereas the discharge conduit for the second effluent is connected with a conduit for the recirculation of the effluent in respectively the one and the other reactor of the first series of reactors, whereby finally the gas holder at the upper side comprises a conduit for recirculating the biogas in the first and second reactor of the first series of reactors and a conduit for discharging the biogas to the consumer.

fig.1

fig.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | BIOTECHNOLOGY AND BIOENGINEERING SYMP., no. 13, 10th-13th May 1984, pages 351-370, John Wiley & Sons, Inc., New York, US; S. GHOSH et al.: "Two-stage upflow anaerobic digestion of concentrated sludge" * Page 353: "Objective"; page 353: "The two-stage upflow digestion concept", line 11 - page 354, line 14 * | 1,2,8 | C 02 F 3/28 |
| A | BIOTECHNOLOGY AND BIOENGINEERING, vol. 25, no. 7, July 1983, pages 1701-1723, John Wiley & Sons, Inc., New York, US; G. LETTINGA et al.: "Anaerobic treatment of raw domestic sewage at ambient temperatures using a granular bed UASB reactor" * Page 1701, "Summary"; page 1705, table II, "Surface load"; page 1707, paragraph "Temperature" * | 2,3,7 | |
| A | EP-A-0 124 409 (L'AIR LIQUIDE) * Page 3, line 21 - page 4, line 14 * | 4,5 | TECHNICAL FIELDS SEARCHED (Int. Cl.4) C 02 F |
| A | KORRESPONDENZ ABWASSER, vol. 30, no. 12, December 1983, pages 904-906, ATV, St. Augustin, DE; T. STEIN et al.: "Die Abwasserreinigung nach dem partiell-aeroben Verfahren der Firma Schering" * Page 904, abstract; page 904, right-hand column, paragraph 4; page 905, right-hand column, paragraph 5; page 906, left-hand column * | 1 | |
| A | DE-C- 666 060 (H. DIECKMANN) * Page 1, line 62 - page 2 * | 1,3,4 | |

The present search report has been drawn up for all claims

| Place of search | Date of completion of the search | Examiner |
|---|---|---|
| THE HAGUE | 01-03-1989 | TEPLY J. |